Europäisches Patentamt
European Patent Office
Office européen des brevets

(11) Publication number: **0 262 893 A2**

(12) **EUROPEAN PATENT APPLICATION**

(21) Application number: **87308553.4**

(51) Int. Cl.$^4$: **A 61 F 9/00**

(22) Date of filing: **28.09.87**

(30) Priority: **02.10.86 GB 8623661**

(43) Date of publication of application: **06.04.88 Bulletin 88/14**

(84) Designated Contracting States: **AT BE CH DE ES FR GB GR IT LI LU NL SE**

(71) Applicant: **Darougar, Sohrab**
**2 Digby Place**
**Croydon, CR0 5QR (GB)**

(72) Inventor: **Darougar, Sohrab**
**2 Digby Place**
**Croydon, CR0 5QR (GB)**

(74) Representative: **Holdcroft, James Gerald, Dr. et al**
**Graham Watt & Co. Riverhead**
**Sevenoaks Kent TN13 2BN (GB)**

(54) **Ocular insert.**

(57) A flexible ocular insert device adapted for the controlled sustained release of an ophthalmic drug into the eye, comprises a body (10) having a circular cylindrical configuration; the length of the device is 8 to 25 mm and the diameter of its body portion 0.5 -1 mm. The sustained release mechanism may be by diffusion through an outer wall of the device, osmosis or bioerosion.

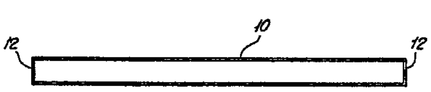


FIG.1

EP 0 262 893 A2

**Description**

"OCULAR INSERT"

This invention is concerned with improvements in or relating to ocular insert devices.

Various diseases of the eye are commonly treated by periodically applying ophthalmic drugs for example in the form of eye drops or ointment. While this is suitable and convenient in some cases, it can be a serious disadvantage that the drug is not dispersed in a continuous manner. With a view to overcoming this disadvantage it has been previously proposed, for example in U.S. Patent 3,416,530 of R.A. Ness assigned to Alza Corporation and subsequent Patents of Alza Corporation to provide a flexible ocular insert device adapted for the controlled sustained release of the drug.

In for example U.S. Patent 3,828,777 again of R.A. Ness assigned to Alza Corporation it is stated that the ocular insert can be fabricated in any convenient shape for comfortable retention in the conjunctival sac of the eye and that the marginal outline can be ellipsoid, doughnut-shape, bean-shape, banana-shape, circular or rectangular; and in cross section it can be doubly convex, concavoconvex, or rectangular. It is suggested however that the original cross-sectional shape of the device is not of controlling importance. However, these previously proposed devices have in practice met with no more than limited success : they have tended not to remain in place in the eye and have at times cause irritation to the patient over the necessary prolonged period of use.

It is an object of the present invention to provide an improved ocular insert device adapted for the controlled sustained release of a drug.

I have found that a flexible ocular insert device having a body of a simple thin elongated circular cylindrical configuration is well retained in place and tolerated by the patient over a prolonged period of use for example up to 7 or 14 days or longer. The insert may be inserted in the upper or lower cul-de-sac of the conjunctiva between the sclera of the eyeball and the upper or lower eyelid, being held in place by the pressure of the lid.

The invention provides in one of its aspects, a flexible ocular insert device adapted for the controlled sustained release of an ophthalmic drug into the eye, characterised in that the device comprises a body having a thin elongated circular cylindrical configuration, the device having for example a length of at least 8 mm and a diameter not exceeding 1 mm.

The circular cylindrical body terminates at transverse end surfaces which may for example be planar or domed.

The material of the insert device is for example a synthetic polymer.

U.S. Patent 4,186,184 of A. Zaffaroni again assigned to Alza Corporation discloses that the length of an insert device should be from 2 to 20 mm, its width 1 to 15 mm and its thickness 0.1 to 4 mm.

I have found however that advantageously the dimensions of a device according to the invention are selected as 8 to 25 mm in length and 0.5 to 1 mm in diameter.

The invention also provides, in another of its aspects a flexible ocular insert device adapted for the controlled sustained release of an ophthalmic drug into the eye, characterised in that the device comprises a body having a circular cylindrical configuration; the length of the device is 8 to 25 mm and the diameter of its body 0.5 to 1 mm.

Examples of ophthalmic drugs include antibiotics such as tetracycline, chlortetracycline, bacitracin, neomycin, polymyxin, gramicidin, cephalexin, oxytetracycline, chloramphenicol, kanamycin, gentamycin, erythromycin and penicillin; antibacterials such as sulfonamides, sulfadiazine, sulfacetamide, sulfamethiazole and sulfisoxazole, nitrofurazone and sodium propionate; antivirals including idoxuridine, trifluorothymidine, acyclovir and interferon; anti-allergenics such as antazoline, methapyriline, chlorpheniramine, and prophenpyridamine; anti-inflammatories such as hydrocortisone, hydrocortisone acetate, dexamethasone, dexamethasone 21-phosphate, fluocinolone, medrysone, prednisolone acetate, fluoromethalone, betamethasone, and triaminolone; decongestants such as phenylphrine, naphazoline and tetrahydrozoline; miotics and antichloinesterase such as pilocarpine, physostigmine, eserine, carbachol, di-isopropyl fluorophosphate, phospholine iodine, and demecarium bromide; mydriatics such as atropine sulfate, cyclopentolate, homatropine, scopolamine, tropicamide, eucatropine, and hydroxyamphetamine; sympathomimetics such as ephinephrine; immunological drugs such as vaccines and immune stimulants; and hormonal agents such as estrogens, estradiol, progestational, progesterone, insulin, calcitonin, parathyroid hormone and peptide, vasopressin, hypothalmus releasing factor; and other drugs such as prostaglandins, antiprostaglandins, and prostaglandin precursors.

The drugs may be used in conjunction with a pharmaceutically acceptable carrier. Examples of pharmaceutically acceptable carriers include solids such as starch, gelatin, sugars, e.g., glucose, natural gums, e.g., acacia, sodium alginate, carboxymethyl cellulose, polymers, e.g., silicone rubber; liquids such as sterile water, saline, dextrose, dextrose in water or saline; condensation products of castor oil and ethylene oxide liquid glyceryl triester of a lower molecular weight fatty acid; lower alkanols; oils such as corn oil, peanut oil, sesame oil, and the like, with emulsifiers such as mono- or di-glyceride of a fatty acid, or a phosphatide, e.g., lecithin, and the like; glycols; polyalkylene glycols; aqueous media in the presence of a suspending agent, for example, sodium carboxymethylcellulose, sodium alginate, poly(vinylpyrolidone), alone, or with suitable dispensing agents such as lecithin, polyoxyethylene stearate. The carrier may also contain adjuvants such as preserving, stabilizing, wetting, emulsifying agents.

The mechanism of controlled sustained drug

release into the eye is for example diffusion, osmosis or bio-erosion and these mechanisms are described for example in the U.S. Patent 4,186,184 and in "Therapeutic Systems" by Klaus Heilmann published by Georg Thieme, Stuttgart 1978.

The period of controlled sustained release is for example up to 7 or 14 days or longer.

In one exemplary embodiment of the present invention utilising the diffusion mechanism the configuration of the insert device is tubular with its cylindrical wall closed by transverse end walls to define a reservoir for the drug which is in liquid or gel form. At least the cylindrical wall is a membrane permeable by diffusion so that the drug is released continuously at a controlled rate through the membrane into the tear fluid.

In one exemplary embodiment of the invention utilising the osmosis mechanism, the configuration of the insert device is tubular with domed end walls, and the device comprises a transverse impermeable elastic membrane dividing the tubular interior of the device into a first compartment and a second compartment; the first compartment is bounded by a semi-permeable membrane and the impermeable elastic membrane, and the second compartment is bounded by an impermeable material and the elastic membrane. There is a drug release aperture in the impermeable end wall of the device.

The first compartment contains a solute which cannot pass through the semi-permeable membrane and the second compartment provides a reservoir for the drug which again is in liquid or gel form.

When the device is placed in the aqueous environment of the eye water diffuses into the first compartment and stretches the elastic membrane to expand the first compartment and contract the second compartment so that the drug is forced through the drug release aperture.

In one exemplary embodiment of the invention utilsing the bioerosion mechanism, the configuration of the insert device is rod-like being constituted from a matrix of bieoerodible material in which the drug is dispersed. Contact of the device with tear fluid results in controlled sustained release of the drug by bioerosion of the matrix. The drug may be dispersed uniformly throughout the matrix but it is believed a more controlled release is obtained if the drug is superficially concentrated in the matrix.

Examples of materials for a permeable membrane for the diffusion mechanism include insoluble microporous materials of polycarbonates, polyvinyl chlorides, polyamides, copolymers of polyvinyl chloride and acrylonitrile, polysulphones, polyvinylidene fluorides, polyvinyl fluorides,polychloroethers, polyformaldehydes, acrylic resins, polyurethanes, polyimides, polybenzimadozoles, polyvinyl acetates, polyethers, cellulose esters, porous rubbers, cross-linked poly(ethylene oxide), cross-linked polyvinyl pyrrolidone, cross-linked poly(vinyl alcohol), polystyrenes.

The drug in liquid or gel form for the diffusion mechanism comprises a diffusion medium which also serves as a pharmaceutical carrier and in which the active ingredient of the drug is dissolved or suspended; the active ingredient is preferably of no more than limited solubility in the medium. Examples of dffusion media include saline, glycerin, ethylene glycol, propylene glycol, water (which may contain also emulsifying and suspending agents), mixtures of propylene glycol monostearate and oils, gum tragacanth, sodium alginate, poly(vinyl pyrrolidone), polyoxyethylene stearate, fatty acids, silicone oil.

Examples of materials for an osmotic semi-permeable membrane include cellulose acetate and its derivatives, partial and completely hydrolyzed ethylene-vinyl acetate copolymers, highly plasticized polyvinyl chloride, homo- and copolymers of polyvinyl acetate, polyesters of acrylic acid and methacrylic acid, polyvinyl alkyl ethers, polyvinyl fluoride; silicone polycarbonates, aromatic nitrogen-containing polymeric membranes, polymeric epoxides, copolymers of an alkylene oxide and alkyl glycidyl ether, polyurethanes, polyclycolic or polylactic acid and derivatives thereof, derivatives of polystyrene such as poly(sodium styrenesulfonate) and poly(vinyl benzyltrimethyl-ammonium chloride), ethylene-vinyl acetate copolymers.

Examples of solutes which cannot pass through the semi-permeable membrane in an osmotic mechanism include water-soluble inorganic and organic salts and compounds such as magnesium sulfate, magnesium chloride, sodium chloride, lithium chloride, potassium sulfate, sodium carbonate, sodium sulfate, lithium sulfate, calcium bicarbonate, sodium sulfate, calcium sulfate, potassium acid phosphate, calcium lactate, magnesium succinate, tartaric acid, acetamide, choline chloride, soluble carbohydrates such as sorbitol, mannitol, raffinose, glucose, sucrose, lactose.

Examples of bioerodible matrix materials include polyesters of the general formula $-O-(W)-CO-$ and mixtures thereof, wherein W is a lower alkylene of 1 to 7 carbons and may include a member selected from the group of alkylenes of the formula $-CH_2-$, or $-CH-CH_2-$, and Y has a value such that the molecular weight of the polymer is from about 4,000 to 100,000. The polymers are polymerization-condensation products of monobasic hydroxy acid of the formula $C_nH_{2n}(OH)COOH$ wherein n has a value of 1 to 7, preferably 1 or 2 and the acid is especially lactic acid or glycolic acid. Also included are copolymers derived from mixtures of these acids. Bioerodible materials also include poly(orthoesters). These materials have the following general formula:

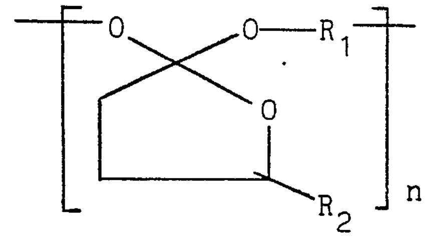

wherein $R_1$ is an alkylene of 4 to 12 carbons, a cycloalkylene of 5 to 6 carbons substituted with an alkylene of 1 to 7 carbons and an alkyleneoxy of 1 to 7 carbons, and $R_2$ is a lower alkyl of 1 to 7

carbons.

Further information on membrane and bioerodible materials is contained in the U.S. Patents 3,828,777 and 4,186,184.

The above and other aspects of the present invention will become more clear from the following description, to be read with reference to the accompanying drawings of devices embodying the invention. This description is given by way of example only, and not by way of limitation of the invention.

In the accompanying drawings:

Figure 1 shows a diagrammatic sectional view of a diffusional ocular insert device embodying the invention;

Figure 2 shows a diagrammatic sectional view of an osmotic ocular insert device embodying the invention; and.

Figure 3 shows an enlarged diagrammatic sectional view of a bioerodible insert device embodying the invention.

The ocular insert device shown in Figure 1 comprises a circular cylindrical wall 10 of a microporous synthetic polymer membrane which is insoluble in tear fluid but is permeable by diffusion. The cylindrical wall 10 is closed by transverse planar end walls 12 which may be of the same microporous synthetic polymer membrane as the cylindrical wall 10 or alternatively may be impermeable. The overall length of the device is 8 to 25 mm and its external diameter 0.5 - 1 mm.

The cylindrical wall 10 and the end walls 12 define a reservoir for a drug which diffuses through the membrane as described hereinbefore.

The ocular insert device shown in Figure 2 comprises a circular cylindrical wall 110 closed by hemispherical domed end portions 112. The device also comprises, perpendicular to the axis of the cylindrical wall, an impermeable elastic membrane 114 dividing the interior of the device into a first compartment 116 and a second compartment 118. The cylindrical wall 110 comprises different materials as respectively do the end walls 112 so that the first compartment is bounded by a semi-permeable synthetic polymer membrane 120 and the elastic membrane 114 and the second compartment is bounded by an impermeable synthetic polymeric membrane 122 and the elastic membrane 114. There is an axial drug release aperture 124 in the membrane 122 at the domed end portion 112 thereof.

The first compartment 116 contains a solute and the second compartment provides a reservoir for a drug which is forced through the aperture 124 by the stretching of the elastic membrane 114 under osmosis as described hereinbefore.

The ocular insert device shown in Figure 3 comprises a circular cylindrical body 210 with domed end portions 212. The device is constituted from a matrix of synthetic polymeric bioerodible material in which a drug is dispersed, being concentrated superficially of the matrix for controlled release therefrom as the matrix bioerodes.

The overall length and diameter of each of the devices of Figrue 2 and Figure 3 is the same as for the device of Figure 1.

**Claims**

1. A flexible ocular insert device adapted for the controlled sustained release of an ophthalmic drug into the eye, characterised in that the device comprises a body (10, 110, 210) having a thin elongated circular cylindrical configuration.

2. A device according to claim 1 having a length of at least 8 mm.

3. A device according to claim 1 having a diameter not exceeding 1 mm.

4. A flexible ocular insert device adapted for the controlled sustained release of an ophthalmic drug into the eye, characterised in that the device comprises a body (10, 110, 210) having a circular cylindrical configuration; the length of the device is 8 to 25 mm and the diameter of its body portions 0.5 -1 mm.

5. A device according to any of the preceding claims wherein the body (10) is tubular and the mechanism of drug release is by diffusion through an outer wall of the device.

6. A device according to any one of claims 1 to 4, wherein the body (110) is tubular and the mechanism of drug release is by osmosis.

7. A device according to any one of claims 1 to 4, wherein the body (210) is rod-like and the mechanism of drug release is bioerosion.

0262893

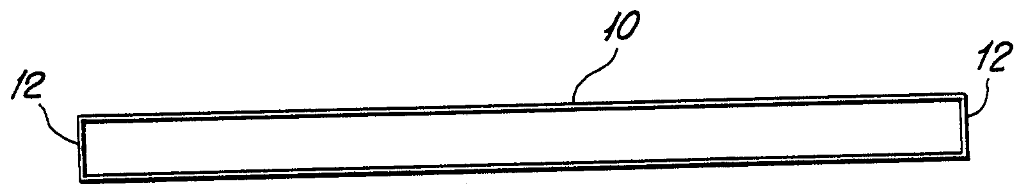


FIG.1

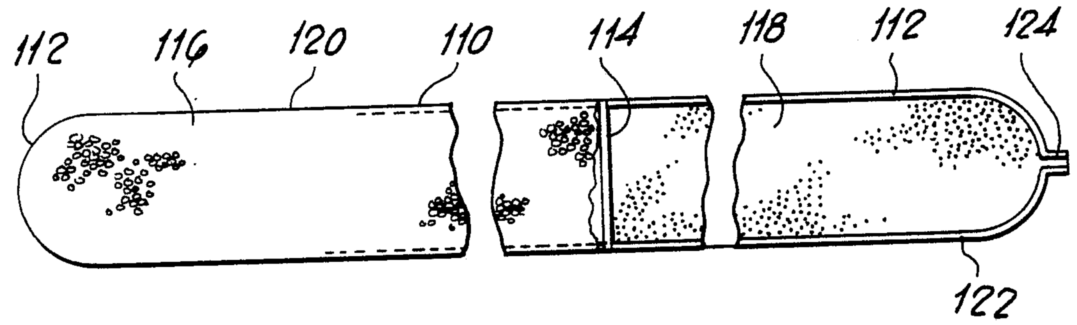


FIG.2

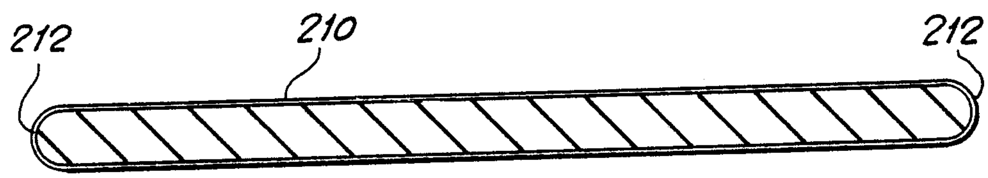


FIG.3